# EUROPEAN PATENT SPECIFICATION

(11) **EP 1 224 211 B1**
(45) Date of publication and mention of the grant of the patent: **16.04.2003**
(21) Application number: 00963143.3
(22) Date of filing: 08.09.2000
(51) Int. Cl.: C07K 5/06

(54) **PROCESS FOR THE PREPARATION OF A DIPEPTIDE AND INTERMEDIATE PRODUCT IN SUCH A PROCESS**
VERFAHREN ZUR HERSTELLUNG EINES DIPEPTIDS UND ZWISCHENVERBINDUNG BEI DIESEM VERFAHREN
PROCEDE DE PREPARATION D'UN DIPEPTIDE ET D'UN PRODUIT INTERMEDIAIRE DANS UN TEL PROCEDE

(30) Priority: 27.10.1999 NL 1013404
(43) Date of publication of application: 24.07.2002
(73) Proprietor: DSM N.V., 6411 TE Heerlen (NL)
(72) Inventor: BOESTEN, Wilhelmus, Hubertus, Joseph, NL-6132 BJ Sittard (NL); BROXTERMAN, Quirinus, Bernardus, NL-6151 JA Sittard (NL); PLAUM, Marcus, Joseph, Maria, NL-6141 AC Limbricht (NL)
(74) Representative: Jacobs, Monique S.N.
(86) International application number: NL0000635
(87) International publication number: WO01030807

(56) References cited:
- EP-A- 0 227 301
- WO-A-96/11209
- WO-A-97/12902

## Description

The invention relates to a process for the preparation of a dipeptide of formula 1 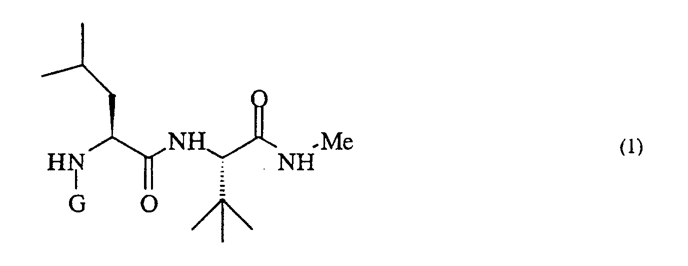 in which G represents a protective group, with N-protected L-leucine being coupled to L-*tert.*-leucine-N-methylamide in the presence of an activating agent.

WO-A-96/11209 discloses such a process in which N-(1,1-dimethylethoxy)carbonyl-L-leucine and L-*tert.*-leucine-N-methylamide are coupled.

A drawback of the known process is that it uses an expensive protective group, so that the process is less attractive from a commercial point of view. The present invention provides a commercially attractive route for the preparation of the above-mentioned intermediate product in the preparation of, for instance, the pharmaceuticals as described in WO-A-96/11209.

This is achieved according to the invention by using a formyl group as protective group.

Dipeptide couplings involving the coupling of two amino acids are generally known and are described in detail in the literature. In these couplings the activated acid group of the eventual N-terminal amino acid reacts with the amino group of the eventual C-terminal amino acid or amino acid derivative. In this process the amino group of the eventual N-terminal amino acid is protected by means of a protective group.

In the process according to the invention two enantiomer-enriched amino acids are coupled. The enantiomeric excess of the enantiomer-enriched amino acids is preferably greater than 80%, in particular greater than 90%, more in particular greater than 98%. It is known that racemization of the N-terminal amino acid may take place when the amino acids are coupled. This is the case in particular when a formyl protective group is used, such as for instance described in the handbooks Houben-Weyl, Band 15/1 (1974), p. 166, and The Peptides, Academic Press 1979, Volume 1, p. 279. As a consequence, formyl protective groups are not considered for coupling of enantiomer-enriched amino acids. Applicant has now found that no racemization or only a low degree of racemization takes place when the coupling is carried out according to the invention, with a formyl group being used as protective group. Moreover, applicant has found that, should racemization take place, this very coupling product according to the invention is particularly suitable for enrichment in the desired diastereomeric form through crystallization.

An added advantage of the process according to the invention is that inexpensive activating agents can be used in the process.

The N-formyl-L-leucine that is used in the process according to the invention can for instance be prepared in a known manner by contacting L-leucine with formic acid and for instance an anhydride. Preferably, use is made of acetic anhydride.

The L-*tert.*-leucine-N-methylamide can for instance be prepared from L-*tert.*-leucine via the conversion of L-*tert.*-leucine and phosgene into L-*tert.*-leucine-N-carboxyanhydride, which is subsequently converted into L-*tert.*-leucine-N-methylamide with the aid of N-methylamine.

In the process according to the invention the N-formyl-L-leucine is activated by means of an activating agent, preferably a sterically hindered acid chloride or an alkyl chloroformiate, and a base. Such activation steps are generally known and are often applied in peptide couplings. The bases to be used therefore are preferably the known bases used in these activation steps, with a low degree of racemization occurring. Preferably, N-methylmorpholine is used as base.

The temperature at which the activation is carried out is not very critical and in practice usually lies between -30°C and +30°C, preferably between -20°C and +10°C.

If desired the activation is carried out in a solvent, preferably one that is inert in the reaction mixture. Examples of solvents esters are esters, in particular ethyl acetate, isopropyl acetate and isobutyl acetate, ethers, in particular tetrahydrofuran (THF), methyl-*tert.*-butylether (MTBE) and dioxane, and nitriles, in particular acetonitrile.

In one embodiment first the activation is carried out followed by a coupling step. For the coupling, the activated N-formyl-L-leucine is contacted with the L-*tert.*-leucine-N-methylamide. Preferably, a solution of L-*tert.*-leucine-N-methylamide is used.

In principle, for the temperature at which the coupling takes place the same holds as for the temperature at which the activation is carried out. Preferably, the coupling temperature is about the same as the activation temperature. Examples of suitable solvents for the L-*tert.*-leucine-N-methylamide are alcohols, in particular methanol, ethanol and isopropanol, esters, in particular ethyl acetate, isopropyl acetate and isobutyl acetate and ethers, in particular THF, MTBE and dioxane.

Alternatively a one stage procedure may be followed for the activation and the coupling, wherein the N-formyl-L-leucine, the L*-tert.*-leucine-N-methyl amide and the base are solved in a suitable solvent as described above, and the activating agent is added to the solution.

The resulting N-formyl-L-leucyl-L*-tert.*-leucine-N-methylamide can subsequently be deformylated in a generally known manner, for instance in an acid environment. The deformylation can for instance be carried out in an aqueous environment, in water/alcohol mixtures or in a two-phase system.

The temperature at which the deformylation is carried out for instance lies between 20°C and 110°C, preferably between 40°C and 80°C.

The resulting N-formyl-L-leucyl-L-*tert.*-leucine-N-methylamide or L-leucyl-L-*tert.*-leucine-N-methylamide can if desired be purified, for instance by subjecting it to a crystallization. Surprisingly, it has been found that the enantiomeric excess of the N-terminal amino acid in the protected or non-protected dipeptide can be increased by the crystallization in those cases in which racemization has taken place during the peptide coupling.

Examples of suitable solvents that can be used in the crystallization are hydrocarbons, in particular heptane and hexane; esters, in particular isopropyl acetate, isobutyl acetate and ethyl acetate; ethers, in particular MTBE; alcohols, in particular methanol, ethanol, isopropanol and butanol; or mixtures thereof. An example of a suitable mixture of solvents is a mixture of heptane and isopropyl acetate.

The temperature at which the crystallization is carried out is not particularly critical and depends mainly on the physical parameters of the chosen solvent, particularly the boiling point. In practice, the crystallization will usually be carried out at a temperature between 20°C and 100°C.

Depending on the exact embodiment of the peptide coupling, it may be advantageous to isolate the N-formyl-L-leucyl-L*-tert.*-leucine-N-methylamide intermediate product obtained, for instance via extraction or crystallization.

The L-leucyl-L-*tert.*-leucine-N-methylamide obtained can for instance be applied in the preparation of pharmaceuticals, for instance the N-(α-optionally substituted mercaptocarboxyl)- L-leucyl-L-*tert.*-leucine-N-methylamide compounds such as described in WO-A-96/11209 and WO-A-97/12902. The α-optionally substituted mercaptocarboxyl group for instance represents a group of formula R₁S-C(R₂)-C(O)- where R₁ stands for H or R₃CO where R₃ is a C₁₋₄ alkyl, (C₁₋₄ alkyl)aryl group, (C₁₋₆ alkyl)heteroaryl group, C₃₋₆ cycloalkyl) group, C₃₋₆ cycloalkyl)C₁₋₄ alkyl group, C₂₋₆ alkenyl group, (C₂₋₆ alkenyl) aryl group, aryl group or heteroaryl group; and R₂ stands for H or a C₁₋₄ alkyl-C(O)-A- or C₁₋₄ alkyl-NH-C(O)-A group, where A stands for 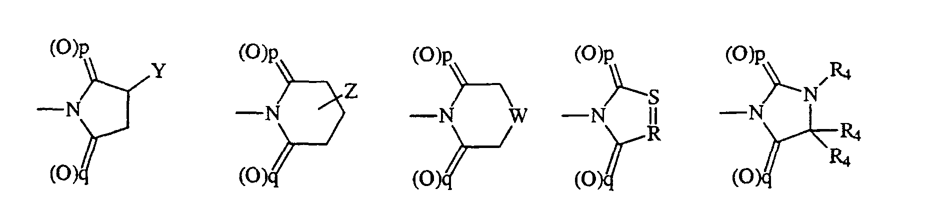 p and q are each independently 0 or 1
R₄ = H or a C₁₋₆ alkyl group (each R₄ independent of the other one)
Y and Z are each independently H or (C₀₋₄ alkyl) R₅, where R₅ is NHR₄, N(R₄)₂ (R₄ each independently), COOR₄, CONHR₄, NHCO₂R₄, NHSO₂R₄ or NHCOR₄ and
W is O, S(O)ₘ, with m = 0, 1 or 2, or NR₆
R₆ = H, C₁₋₄ alkyl, COR₇, CO₂R₇, CONHR₇ or SO₂R₇
R₇ = H, C₁₋₄ alkyl, aryl, heteroaryl, (C₁₋₄ alkyl)aryl or (C₁₋₄ alkyl) heteroaryl
R and S are each independently CH or N.

These compounds can be prepared in a known manner by for instance activating a substituted or non-substituted α-mercaptocarboxylic acid and coupling it to the L-leucyl-L-*tert.*-leucine-N-methylamide dipeptide obtained according to the invention using classical peptide coupling techniques, as for instance described in WO-A-96/11209 and WO-A-97/12902.

The invention will now be elucidated on the basis of examples, without however being restricted thereto.

### Example I

### Preparation of N-formyl-L-leucyl-L-tert.-leucine-N-methylamide from N-formyl-L-leucine and L-tert.-leucine-N-methylamide

Under nitrogen at -18°C isobutylchloroformiate (6.5 g, 48 mmol) was dosed to a solution of N-formyl-L-leucine (8.0 g, 50 mmol) in tetrahydrofuran (125 ml). Then N-methyl morpholine (4.8 g, 48 mmol) was added dropwise at such a rate that the temperature remained < -15°C. A precipitate was formed.

After stirring had been continued for 15 minutes, a solution of L-*tert.*-leucine-N-methylamide (6.5 g, 45 mmol) in tetrahydrofuran (50 ml) was added in such a way that the temperature remained < -15°C. Subsequently, stirring was continued for 1 hour at-18°C.

The reaction mixture was heated to 0°C and at this temperature water was added (100 g). Then THF was removed by distillation under vacuum. Isopropyl acetate (75 ml) was added and the pH of the reaction mixture was adjusted to 1.5 using hydrochloric acid. After layer separation, the aqueous phase was twice extracted with 50 and 35 ml isopropyl acetate, respectively. The collected organic phases were then washed with 50 and 25 ml saturated sodium bicarbonate solution and finally with 25 ml water. The organic phase was then evaporated under vacuum.

N-formyl-L-leucyl-L*-tert.*-leucine-N-methylamide was obtained in a good yield and with an e.e. (L-leucine fragment) of 99% (HPLC).

### Example II

### Preparation of L-leucyl-L-tert.-leucine-N-methylamide from N-formyl-L-leucyl-L-tert.-leucine-N-methylamide

11.7 g (41 mmol) N-formyl-L-leucyl-L-*tert.*-leucine-N-methylamide (see Example I) was suspended in 1M HCl (100 ml) and heated to 40°C. After 18 hours' stirring at this temperature (all material went into solution), cooling to room temperature and one extraction with 50 ml isopropyl acetate took place.

After layer separation the pH of the aqueous phase was adjusted to 10 using 50% sodium hydroxide solution. Two extractions with isopropyl acetate (75 ml) were performed. The collected organic phases were evaporated under vacuum.

The residue was suspended in heptane (75 ml) and heated to 65°C. So much isopropyl acetate was added that everything just dissolved. After crystallization by means of cooling to room temperature and filtration, the material was washed twice with heptane (25 ml) and dried. L-leucyl-L-*tert.*-leucine-N-methylamide was obtained in a good yield with
purity = >98% (HPLC)
e.e. (L-leucine fragment) = 99% (HPLC)

### Example III

### Preparation of N-formyl-L-leucyl-L-tert.-leucine-N-methylamide from N-formyl-L-leucine and L-tert.-leucine-N-methylamide

Under nitrogen at -15°C isobutylchloroformiate (12.3 g, 90 mmol) was dosed to a suspension of N-formyl-L-leucine (15.9 g, 100 mmol) in isopropyl acetate (85 ml). Subsequently, N-methyl morpholine (9.1 g, 90 mmol) in isopropylacetate (25ml) was added dropwise at such a rate that the temperature remained < -10°C.

After stirring had been continued for 90 minutes, the suspension formed was dosed to a cooled solution of L-*tert.*-leucine-N-methylamide (13.0 g, 90 mmol) in methanol (65 ml) in such a way that the temperature remained < -10°C. Stirring was subsequently continued for 30 minutes at -10°C.

The reaction mixture was heated to room temperature and further stirred at this temperature for 2 hours. 100 ml water was added to the reaction mixture and the pH was adjusted to 1.0 using 37% aqueous hydrochloride solution. After layer separation the aqueous phase was rewashed with two times 75 ml isopropyl acetate. The collected organic phases were then washed with 100 and 50 ml saturated sodium carbonate solution, respectively.

The organic phase was then evaporated under vacuum. N-formyl-L-leucyl-L-*tert.*-leucine-N-methylamide was obtained with an e.e. (L-leucine fragment) of 98% (HPLC).

### Example IV

### Preparation of N-formyl-L-leucyl-L-tert.-leucine-N-methylamide from N-formyl-L-leucine and L-tert.-leucine-N-methylamide

N-formyl-L-leucyl-L*-tert.*-leucine-N-methylamide was prepared as described in Example III, but now at temperatures between 0-5°C. N-formyl-L-leucyl-L*-tert.*-leucine-N-methylamide was obtained with an e.e. (L-leucine fragment) of 86% (HPLC).

### Example V

### Preparation of L-leucyl-L-tert.-leucine-N-methylamide from N-formyl-L-leucyl-L-tert.-leucine-N-methylamide

The material obtained in Example IV was treated as described in Example II. L-leucyl-L-*tert.*-leucine-N-methylamide was obtained with an e.e. (L-leucine fragment) of 95% (HPLC).

## Claims

1. Process for the preparation of a dipeptide of formula 1 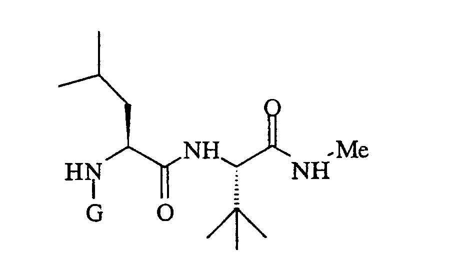 where G represents a protective group with N-protected L-leucine being coupled to L-*tert.*-leucine-N-methylamide in the presence of an activating agent, **characterized in that** a formyl group is used as protective group.

2. Process according to claim 1 in which the L-*tert.*-leucine-N-methylamide has an enantiomeric excess greater than 98%

3. Process according to claim 1 or 2 in which the N-formyl-L-leucine has an enantiomeric excess greater than 98%.

4. Process according to any one of claims 1-3 in which the N-formyl-L-leucyl-L-*tert.*-leucine-N-methylamide obtained is subsequently subjected to one or more crystallizations.

5. Process according to any one of claims 1-4 in which the dipeptide obtained is subsequently deformylated.

6. Process according to claim 5 in which the L-leucyl-L-*tert.*-leucine-N-methylamide obtained is subsequently subjected to one or more crystallizations.

7. Process according to claim 5 or 6 in which the L-leucyl-L-*tert.*-leucine-N-methylamide is subsequently coupled to a substituted or non-substituted α-mercaptocarboxylic acid to form the corresponding N-α-optionally substituted mercaptocarboxyl-L-leucyl-L-*tert.*-leucine-N-methylamide.

8. N-formyl-L-leucyl-L-*tert.*-leucine-N-methylamide.

9. N-formyl-L-leucyl-L-*tert.*-leucine-N-methylamide with an enantiomeric excess of the N-terminal amino acid in the dipeptide of more than 80%.

10. N-formyl-L-leucyl-L-*tert.*-leucine-N-methylamide with an enantiomeric excess of the N-terminal amino acid in the dipeptide of more than 98%.

11. N-formyl-L-leucyl-L-*tert.*-leucine-N-methylamide according to claim 9 or 10 with a diastereomeric excess of more than 80%.

12. N-formyl-L-leucyl-L-*tert.*-leucine-N-methylamide according to claim 11 with a diastereomeric excess of more than 98%.

13. Use of N-formyl-L-leucyl-L-*tert.*-leucine-N-methylamide according to any one of claims 8-12 in the preparation of pharmaceuticals.

## Patentansprüche

1. Verfahren für die Herstellung eines Dipeptids der Formel 1 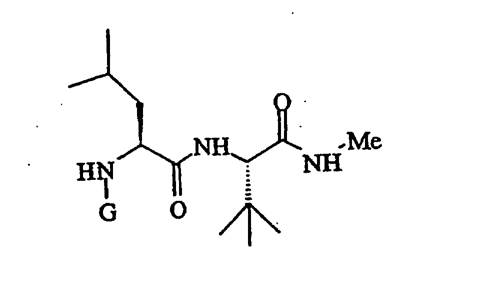 worin G eine Schutzgruppe darstellt, wobei N-geschütztes L-Leucin in Gegenwart eines Aktivierungsmittels an L-tert.-Leucin-N-methylamid gekoppelt wird, **dadurch gekennzeichnet, dass** eine Formylgruppe als Schutzgruppe verwendet wird.

2. Verfahren gemäß Anspruch 1, wobei das L-tert.-Leucin-N-methylamid einen enantiomeren Überschuss größer als 98% hat.

3. Verfahren gemäß Anspruch 1 oder 2, wobei das N-Formyl-Lleucin einen enantiomeren Überschuss größer als 98% hat.

4. Verfahren gemäß einem der Ansprüche 1-3, wobei das erhaltene N-Formyl-L-leucyl-L-tert.-leucin-N-methylamid nachfolgend einer oder mehreren Kristallisationen unterworfen wird.

5. Verfahren gemäß einem der Ansprüche 1-4, wobei das erhaltene Dipeptid nachfolgend deformyliert wird.

6. Verfahren gemäß Anspruch 5, wobei das erhaltene L-Leucyl-Ltert.-leucin-N-methylamid nachfolgend einer oder mehreren Kristallisationen unterworfen wird.

7. Verfahren gemäß Anspruch 5 oder 6, wobei das L-Leucyl-Ltert.-leucin-N-methylamid nachfolgend an eine substituierte oder nicht substituierte α-Mercaptocarbonsäure gekoppelt wird, um das entsprechende N-α-gegebenenfalls substituierte Mercaptocarboxyl-L-leucyl-L-tert.-leucin-N-methylamid zu bilden.

8. N-Formyl-L-leucyl-L-tert.-leucin-N-methylamid.

9. N-Formyl-L-leucyl-L-tert.-leucin-N-methylamid mit einem enantiomeren Überschuss der N-endständigen Aminosäure im Dipeptid von mehr als 80%.

10. N-Formyl-L-leucyl-L-tert.-leucin-N-methylamid mit einem enantiomeren Überschuss der N-endständigen Aminosäure im Dipeptid von mehr als 98%.

11. N-Formyl-L-leucyl-L-tert.-leucin-N-methylamid gemäß Anspruch 9 oder 10 mit einem diastereomeren Überschuss von mehr als 80%.

12. N-Formyl-L-leucyl-L-tert.-leucin-N-methylamid gemäß Anspruch 11, mit einem diastereomeren Überschuss von mehr als 98%.

13. Verwendung von N-Formyl-L-leucyl-L-tert.-leucin-N-methylamid gemäß einem der Ansprüche 8-12 bei der Herstellung von Pharmazeutika.

## Revendications

1. Procédé pour la préparation d'un dipeptide de formule 1 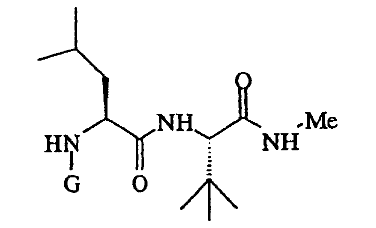 où G représente un groupe protecteur, dans lequel une L-leucine N-protégée est couplée au N-méthylamide de la L-tert-leucine en présence d'un agent activateur, **caractérisé en ce qu'**un groupe formyle est utilisé comme groupe protecteur.

2. Procédé selon la revendication 1, dans lequel le N-méthylamide de la L-tert-leucine a un excès énantiomèrique supérieur à 98 %.

3. Procédé selon la revendication 1 ou 2, dans lequel la N-formyl-L-leucine a un excès énantiomèrique supérieur à 98 %.

4. Procédé selon l'une quelconque des revendications 1 à 3, dans lequel le N-méthylamide de la N-formyl-L-leucyl-L-tert-leucine obtenu est ensuite soumis à une ou plusieurs cristallisations.

5. Procédé selon l'une quelconque des revendications 1 à 4, dans lequel le dipeptide obtenu est ensuite déformylé.

6. Procédé selon la revendication 5, dans lequel le N-méthylamide de la L-leucyl-L-tert-leucine obtenu est ensuite soumis à une ou plusieurs cristallisations.

7. Procédé selon la revendication 5 ou 6, dans lequel le N-méthylamide de la L-leucyl-L-tert-leucine obtenu est ensuite couplé à un acide α-mercaptocarboxylique substitué ou non-substitué pour former le N-méthylamide de la N-α-mercaptocarboxyl(éventuellement substitué)-L-leucyl-L-tert-leucine.

8. N-méthylamide de la N-formyl-L-leucyl-L-tert-leucine.

9. N-méthylamide de la N-formyl-L-leucyl-L-tert-leucine avec un excès énantiomèrique de l'acide aminé N-terminal dans le dipeptide supérieur à 80 %.

10. N-méthylamide de la N-formyl-L-leucyl-L-tert-leucine avec un excès énantiomèrique de l'acide aminé N-terminal dans le dipeptide supérieur à 98 %.

11. N-méthylamide de la N-formyl-L-leucyl-L-tert-leucine selon la revendication 9 ou 10, avec un excès diastéréoisomèrique supérieur à 80 %.

12. N-méthylamide de la N-formyl-L-leucyl-L-tert-leucine selon la revendication 11, avec un excès diastéréoisomèrique supérieur à 98 %.

13. Utilisation du N-méthylamide de la N-formyl-L-leucyl-L-tert-leucine selon l'une quelconque des revendications 8 à 12, dans la préparation de produits pharmaceutiques.
